Europäisches Patentamt

European Patent Office

Office européen des brevets

(11) Publication number: **0 171 000**
A2

# EUROPEAN PATENT APPLICATION

(21) Application number: **85109405.2**

(22) Date of filing: **26.07.85**

(51) Int. Cl.⁴: **C 12 N 15/00**, C 12 P 21/02, C 12 N 11/04, C 12 N 11/10

(30) Priority: **27.07.84 JP 157038/84**

(43) Date of publication of application: **12.02.86 Bulletin 86/7**

(84) Designated Contracting States: **AT BE CH DE FR GB IT LI LU NL SE**

(71) Applicant: **Suntory Limited, 1-40 Dojimahama 2-chome Kita-ku, Osaka-shi Osaka-fu 530 (JP)**

(72) Inventor: **Oshima, Takehiro, 1-1, Wakayamadai 1-chome Shimamoto-cho, Mishima-gun Osaka (JP)**
Inventor: **Tanaka, Shoji, 1-1, Wakayamadai 1-chome Shimamoto-cho, Mishima-gun Osaka (JP)**
Inventor: **Tsujimoto, Masafumi, 1-1, Wakayamadai 1-chome Shimamoto-cho, Mishima-gun Osaka (JP)**
Inventor: **Nakazato, Hiroshi, 1-1, Wakayamadai 1-chome Shimamoto-cho, Mishima-gun Osaka (JP)**

(74) Representative: **Eitle, Werner, Dipl.-Ing. et al, Hoffmann, Eitle & Partner Patentanwälte Arabellastrasse 4, D-8000 München 81 (DE)**

(54) Process for producing heterologous matured protein or peptide.

(57)    A method of producing a desired protein or peptide such as interleukin 2, α-neoendorphin, etc., by using recombinant DNA technology is disclosed. A secretor expression vector which is useful in this invention is constructed by replacing a part or whole of a DNA sequence coding for a matured α-factor peptide in an α-factor operon of yeast by a DNA sequence coding for the desired protein or peptide, and optionally exchanging a promoter of the – factor for another promoter derived from yeast. By using the secretory expression vector, yeast is transformed. The transformant is immobilized onto a carrier gel, and cultured, and the desired product is then isolated from the culture medium.

42 441m/ae
0171000

# PROCESS FOR PRODUCING HETEROLOGOUS
## MATURED PROTEIN OR PEPTIDE

This invention relates to a method of producing a matured protein or peptide. In particular, this invention relates to a method of producinng a matured protein or a matured peptide which comprises transforming yeast with an expression vector capable of extracellularly secreting a heterologous protein or peptide produced in yeast cells; immobilizing the yeast transformant onto a carrier gel, culturing the immobilized transformant yeast, and isolating and purifying the matured protein or peptide from the culture solution.

In recent years, attempts have been made to produce in a large amount an effective physiological active substance originating from humans or animals by using recombinant DNA technology. However, there are scarcely any production examples which use a vector capable of secreting a heterologous substance (protein or peptide) produced in a cell outwardly thereof, particularly into a culture medium. It is extremely important in practice to secretively produce the desired heterologous protein or peptide outward the cell (in a periplasm or a medium) because of the small amount of impurities involved when purifying the substance.

Some examples of proteins or peptides which have been secretively produced from a yeast outwardly of the cell have been reported. Substances secreting into a periplasm include as representatives an acid phosphatase and an invertase. A N-glycosylation may be recognized in any of these enzyme proteins.

On the other hand, α-factor (MFα), a-factor and a killer factor are known as substances which may be secreted into a medium.

The present inventors paid attention to these proteins and peptides secretively produced from a yeast and investigated the production of an expression vector for secretively producing a heterologous protein or peptide outside the yeast cell by employing a DNA sequence coding for the genes of these substances.

The mechanisms of the secretor expression in a natural yeast are broadly classified into two groups.

The first is a secretion by a signal peptide. As a signal peptide, the substances employed are derived from an acid phosphatase, and the signal peptide is cleaved when passing through the inner cell membrane. There have been some reports about the secretive production of, for example, a human interferon with use of a signal peptide derived from an animal cell [Science, 219, 620 - 625 (1983)]. According to these reports, the secretive activity is 10 - 30% relative expression is recognized, the processing of the signal peptide is not always brought about at a correct site, causing the processing of about half the molecules to be brought about at different sites. Therefore, this secretion is not said to be practical with regard to the production of a single substance.

The second case is accompanied with the particular processing shown in the secretion of MFα. In this case, the precursor of the precursor of MFα (165 amino acid residues) is first cleaved at the site of Lys-Arg and then the precursor MFα containing 17 or 19 additional amino acid residues is produced. Then the peptide composed of repeating Glu-Ala two or three times, which is bonded to the N-terminal, is subjected to digestion with diaminopeptidase to release a matured MF into a culture medium [Julius, D., etc., Cell, 32, 839 - 852 (1983)].

The present inventors investigated the possibility of production of a secretor expression vector which employs the latter secretory system and thereby found that this system could be appropriately applied to the secretor expression of a heterologous protein or peptide, resulting in the completion of the present invention. The advantages of the secretor expression vector constructed by employing this system are as follows:

(1)     The desired peptide can be stably produced even in a case where it has a small molecular weight.

(2) The production of the desired protein or peptide has no affect on its amino acid composition. The generally used method in which an expressed protein or peptide is treated by CNBr (for example, insulin A chain, insulin B chain, somatostatin and α-neoendorphin) cannot be applied if a methionine residue is present in the desired substance which means that the species of substances obtainable is limited. However, in the system employed in the present invention, there is no such limitation.

(3) The addition of a sugar chain to the desired substance (protein or peptide) can be expected during the secretion process.

(4) Since the protein or peptide containing the desired substance produced by the secretor expression vector is subjected to special processing, no f-Met (or Met) is added to a N-terminal which, if it occurred, would become a problem in the direct expression. Furthermore, the extracellular secretion of the desired substance gives the following advantages:

(5) It is even possible to produce a desired substance which is harmful to a host cell because of the gradual release thereof from cells.

(6) A contineous culture is possible and purification of the desired products is easy.

(7) It is possible to culture a host yeast in an immobilized state and to simplify the production process.

The present invention, having these numerous advantages, will now be explained with reference to the production of α-neoendorphin (αNE), which is a peptide hormone, as a heterologous peptide; and of interleukin 2(IL2) which is substance related to immunity. However, the present invention can be applied to the production of many other useful proteins or peptides, for example, insulin, somatostatine, growth hormone, growth hormone-stimulating factor, and diuretic hormone which are peptide-hormones of humans and mammals; γ-type interferon, tumor necrosis factor (TNF) and lymphotoxin and various interferons having anti-virus activities which are immune-related substances.

In the present invention, the DNA sequence coding for the desired substance, which is used for constructing a secretor expression vector in a host yeast, may be selected from chemically synthesized DNA sequences, complementary DNA obtained by a reverse transcriptase from mRNA, and DNA sequences obtained from chromosomes by suitable treatment, depending on the case.

The secretor expression vector in the present invention is constructed by inserting a DNA sequence coding for the desired substance in the place of all or a part of a DNA sequence coding for a matured MF$\alpha$ peptide in MF$\alpha$ operon. That is to say, the DNA sequence coding for the desired substance is joined to a site downstream from the DNA sequence coding for the signal peptide of MF$\alpha$ operon, a poly-peptide capable of having a sugar chain added to it, and a peptide containing a structure wherein Glu-Ala is repeated two to three times and containing Lys-Arg (which it is possible to remove). A promoter for expressing such a fused gene is selected from a promoter of MF$\alpha$ operon described in the Examples hereunder, a promoter of another yeast having a strong activity, for example, a promoter of the gene controlling the glycolytic pathway enzyme of an yeast such as glyceraldehyde-3-phosphodehydrogenase (GAP-DH), 3-phosphoglycerokinase (PGK), hexokinase, and a promoter for the gene controlling an inducibly expressed enzyme such as a repressible acid phosphatase (for example, PH05).

As described above, since the desired substance (protein or peptide) can be secretively produced in the medium by culturing the yeast which has been transformed by the vector constructed according to this invention, it is possible to continuously produce the desired substance by immobilizing transformant cells on a suitable carrier and culturing them. Furthermore, it is possible according to this invention to produce proteins or peptides with sugar chains by using the secretor expression vector of the present invention.

Thus, the present invention provides a method for efficiently producing heterologous proteins or peptides in large amounts.

Fig. 1 shows the scheme for constructing a plasmid pYMF88 by inserting the DNA fragment of about 1.4 Kb which had been obtained by digesting all of the DNA of yeast X2180'-1B wth EcoRI/SalI into the EcoRI-SalI DNA fragment of pBR322.

Figs. 2-1 and 2-2 show the scheme for constructing MFα gene expression vector pYE2411 by inserting MF$^\alpha$ gene into an yeast-_Escherichia_ _coli_ shuttle vector.

Fig. 3 shows the base sequence of chemically synthesized Met-αNE gene.

Fig. 4 shows the scheme for constructing αNE expression plasmid vectors of pYαNE412, pYαNE413, pYαNE414 and pYαNE415 from pYE2411, pYMF88 and chemically synthesized Met-αNE gene.

Fig. 5 is a graph showing the change with the passage of time of intra- and extracellular expressions of αNE by the transformed yeast with pY NE412.

Fig. 6 shows the base sequence of IL2 gene designed for expression in yeast and chemically synthesized.

Fig. 7 shows the base sequences of 56 chemically synthesized DNA fragments used for constructing IL2 gene.

Fig. 8 explains the ligation strategy for constructing IL2 gene from the DNA fragments in Fig. 7.

Fig. 9 shows the scheme for constructing the IL2 expression vector pS120 in which IL2 gene was inserted in the place of a γIFN gene of a plasmid vector pS20 which contains γIFN gene and bears $\lambda P_L$ promoter and the SD region of $\lambda C_{II}$ protein gene.

Fig. 10 shows the scheme for constructing secretor expression vector pYIL241 for yeast host from pS120, pBR322 and pYMF88.

Fig. 11 is a photograph which shows paterns of SDS-PAGE of the culture supernatant by yeast XS16-1B[pYIL241] transformed with vector pYIL241.

Fig. 12 is a graph showing the change with passage of time of IL2 activity in the culture medium of transformed yeast XS16-1B [pYIL241].

Fig. 13 is a graph showing the dependency of the 3H-thymidine uptake in mouse CTLL-2 cells upon the IL2 concentration.

Fig. 14 is a graph showing the proliferation behavior of the IL2-dependent mouse CTLL cell produced in the present invention.

Fig. 15 is a graph showing the fractionation of crude IL2 of the present invention by chromatofocusing.

Fig. 16 is a graph showing the fractionation of crude IL2 of the present invention by a Con-A Sepharose column.

Fig. 17 is a graph showing the fractionation of crude IL2 of the present invention by an ion exchange column.

Fig. 18 is a graph showing the fractionation of crude Il2 of the present invention by a hydrophobic chromatograpy.

Fig. 19 is a graph showing the change with passage of time of secretor expression of IL2 of αNE produced by immobilizing the transformed yeast produced by the present invention.

An α-factor (MFα) gene was isolated and its structure was reported by J. Kurjan and I. Herskowitz (cedd, 30, 933 - 943) in 1982.  According to this reference, four DNA sequences coding for the matured α-factor, each of which is composed of 13 amino acid residues, are lined in tandem through DNA sequences coding for special spacer peptides. The present inventors therefore deduced that proteins or peptides coded with a foreign heterologous gene can be extracellularly produced by a host yeast if the heterologous gene is inserted to exchange for all of or a part of the DNA sequence coding for the matured MFα peptide.  According to Kurjan, et al. described above, since the MFα operon (gene) is present in the EcoRI fragment (1.7 Kb) of a yeast chromosome and there is a SalI cutting site at 36bp downstream from the termination codon of the MFα gene, it is to be expected that the MFα gene is carried on the EcoRI-SalI fragment of about 1.4 Kb.  Therefore, all the DNA components were isolated from the α-mating type strain X2180-1B yeast (MATα  SUC2 mal gall2 CUPl:  yeast genetic stock center;

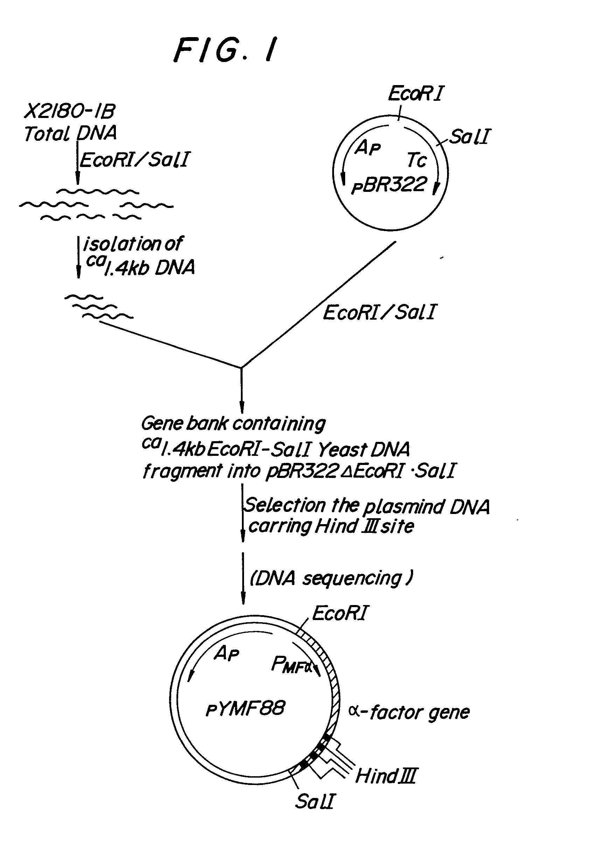

available from University of California, Berkley School, CA 94720), purified and then digested by using EcoRI and SalI to collect DNA fragments of about 1.4 Kb. The DNA fragment was jointed to a DNA sequence containing β-lactamase gene (Amp$^r$) and a replicon obtained from pBR322 by digestion with EcoRI and SalI and then Escherichia coli (JA221) is transformed with the constructed plasmid. Plasmid DNAs were separated from the transformant having an ampicillin resistance by a convenient method, and a plasmid which will form fragments of about 1.2 - 1.3 Kb by digestion with EcoRI and HindIII was selected. The salI cutting site of this plasmid was labelled with $^{32}$P and the DNA base sequence was determined by the MAXAM-GILBERT method to confirm that the plasmid pYMF88 containing the DNA fragment equal to the MFα gene obtained by Kurjan et al. was obtained (Fig. 1). The Escherichia coli transformed by this plasmid (JA221/pYMF88) is called SBM276 and is deposited with Fermentation Research Institute, Japan (deposit No.: FERM P-7735; International Deposition Acceptance No. FERM BP-837 under the Budapest Treaty).

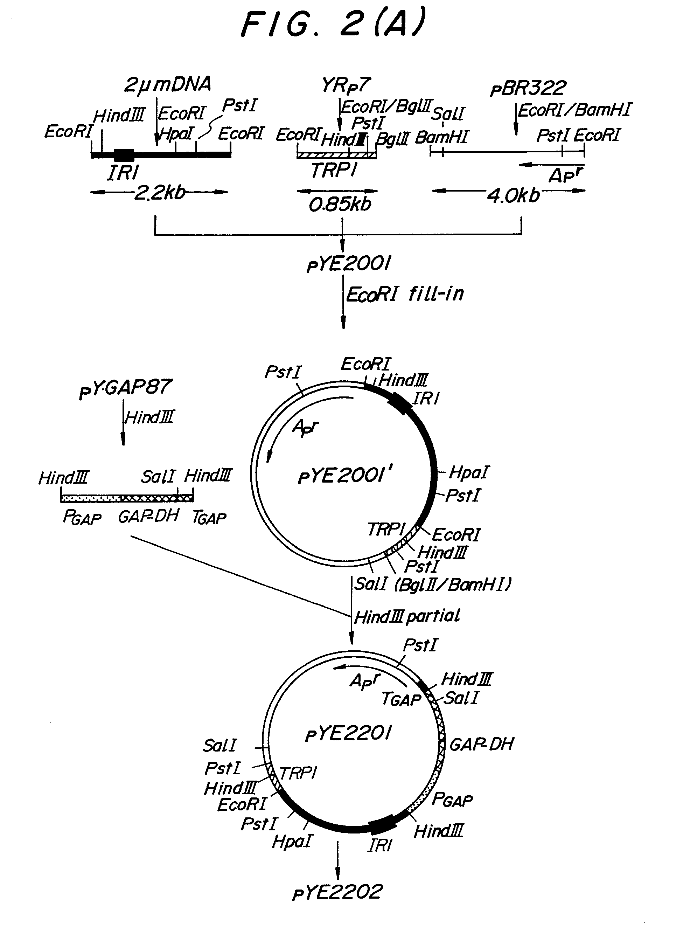

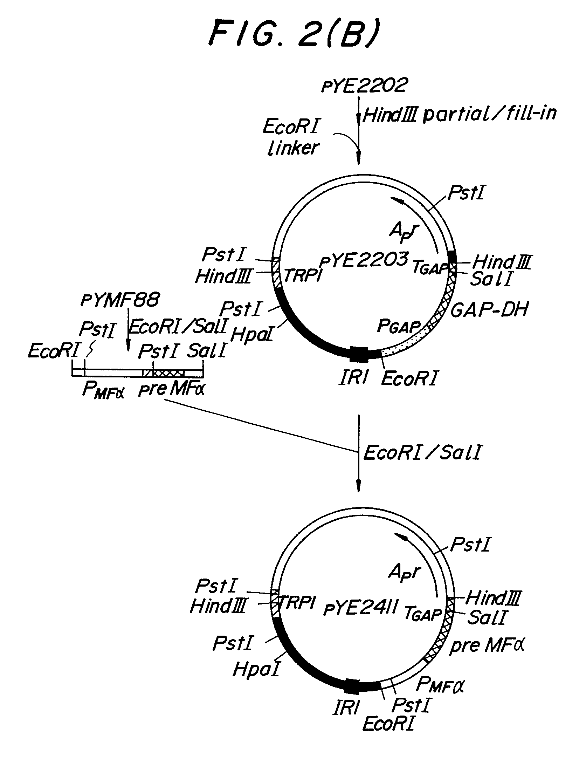

On the other hand, a secretor vector for expression of an heterologous foreign gene (pYE2411) was constructed according to the process shown in Fig. 2. A Shuttle vector PYE2001' is first formed from a yeast of 2 μm DNA, plasmid YR$_p$7 containing the TRP$_1$ gene and pBR322 through pYE2001, and then the Hind III fragment of plasmid pYGAP87 (refer to Japanese Patent Public Disclosure No. 74986/1984: Escherichia coli containing pYGAP87 in which a yeast GAP-DH gene has been cloned was called SMB151 and given International Deposition Acceptance No. FERM BP-382 by Fermentation Research Institute, Japan) was inserted into the HindIII site near IR1 of pYE2001' (pYE2201). Subsequently, yYE2203 was constructed by the process shown in Fig. 2 through pYE2202 and the EcoRI-SalI fragment containing the MFα gene of pYMF88 previously cloned was inserted in the place of the EcoRI-SalI fragment containing GAP-DH gene region (not containing a 3'-terminal non-translation region TGAP of GAP-DH gene) on pYE2203 to obtain a shuttle vector pYE2411 for secretor expression of the desired heterologous protein (or peptide). The plasmid is one produced so as to express a heterologous, exogenous gene under the control of the promoter of MFα gene and to add 3'-terminal non-translation region ($T_{GAP}$) of GAP-DH gene to the downstream of a heterologous exogenous gene. Furthermore, <u>Escherichia</u> <u>coli</u> transformed by pYE2411 is called SMB275 and is deposited with Fermentation Research Institute (Deposition No.: FERM P-7728; International Deposition Acceptance No. FERM BP-836).

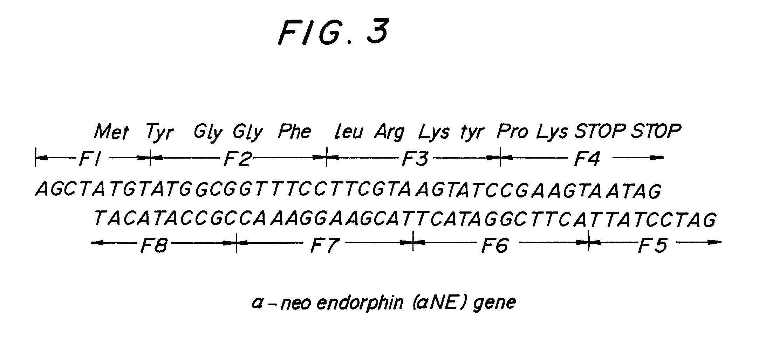

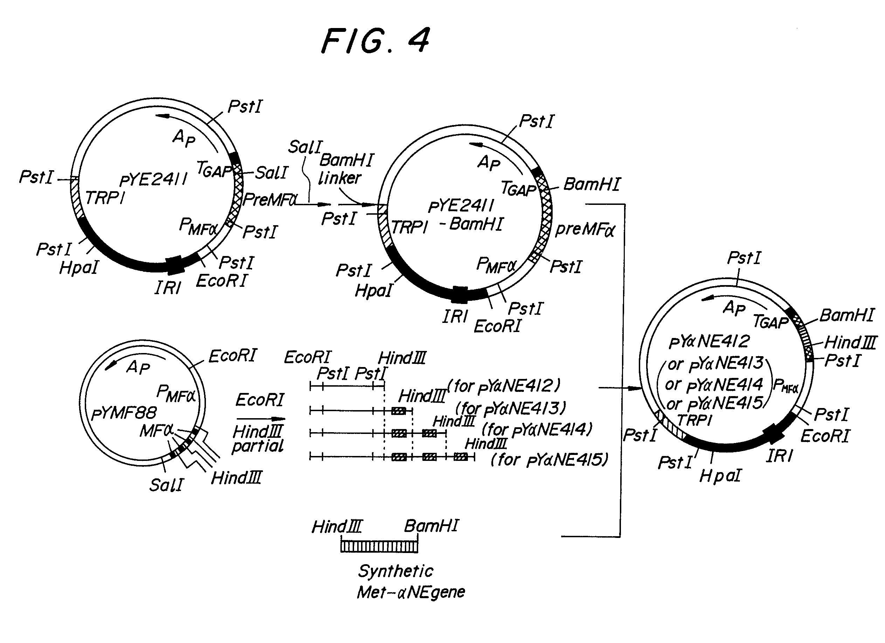

The capability of secretor expression of an exogenous gene was then investigated. First, the DNA sequence coding for α-neoendorphine (αNE) (10 amino acid residues) was used as a physiological active peptide having a molecular size nearly equal to the matured MFα peptide for the purposes of the investigation. Furthermore, the DNA sequence coding for the Met-αNE (11 amino acid residues), in which one methionine residue was added to the N-terminal of αNE in order to investigate the chemical process by CNBr, was exchanged for the DNA sequence coding for the matured MFα peptide so as to investigate the expression in yeast cells. Each one of four matured MFα DNA sequences was exchanged for the Met-α NE gene to construct four plasmids of pY NE412, pYα NE413, pYα NE 414 and pYα NE415 (Fig. 4). The αNE gene used was one in which the cohesive ends of HindIII and BamHI were joined by T4 DNA ligase to the terminals of the DNA sequence chemically synthesized by the triester method (Fig. 3). The plasmids obtained in such a manner are pYα NE412, pYαNE413, pYαNE141 and pYαNE415 which are expressed to produce a protein which does not contain the matured MFα and is composed of 100 amino acid residues: a protein containing one molecule of the matured MFα and composed of 121 amino acid residues; a protein containing 2 molecules of the matured MFα and composed of 142 amino acid residues; and a protein containing 3 molecules of the matured MFα and composed of 162 amino acid residues, respectively. These plasmids are transformed into suitable host yeasts, for example, α-mating type of saccharmyces yeast (<u>Saccharomyces</u> <u>cerevisiae</u> XS16-1B MATα lue2 trpl his3). When the transformant obtained is cultured in a suitable medium, the desired substance $\alpha$NE is found to be secreted. The $Trp^+$ transformant is selected from the special transformants described above, and is cultured in a minimal medium containing 2% Casamino acid (0.67% Difco Nitrogen base, 2% glucose) at 30°C and then the cells and the supernatant liquid are separated. The $\alpha$NE content of each of them can be measured to determine the productivity.

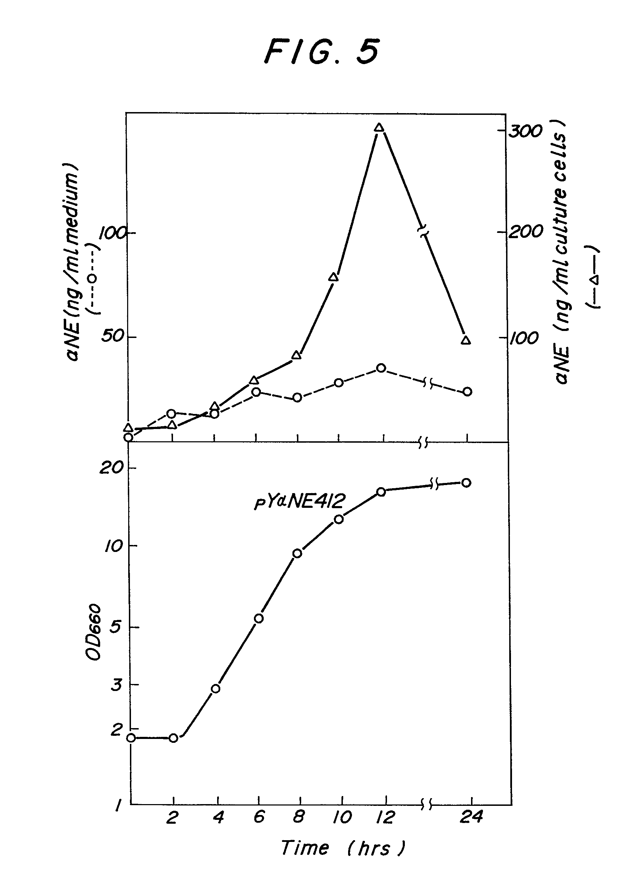

As will be explained in detail by the Examples hereunder, the secretor expression of $\alpha$NE was observed for any plasmid replaced with any of the above-explained part of the DNA sequences coding for the matured MF$\alpha$ as shown in Table 1. In the cell growth phase of the transformed yeast (9.5 hours: logarithmic growth metaphase), 19 - 38% of the total of $\alpha$NE produced is secreted. However, the secretory ratio in a stationary phase (24 hours) is as low as 3.3 - 11%. Furthermore, the time-dependent changes of the $\alpha$NE amounts produced in the cells and in the culture medium are shown in Fig. 5. The $\alpha$NE amount produced in the cells increases in proportion to the cell growth but its accumulation is observed even after the proliferation rate began to decrease (after 8 hours). This is believed to be caused by the degree of secretion, which does not correspond to the expressed amount. On the other hand, the $\alpha$NE amount in the culture medium is not found to change after 6 hours, which is thought to be attributable to the decomposition of the secreted $\alpha$NE in the medium.

The secretion amount is not said to be sufficient, but these characteristics are suitable for continuously culturing the transformed yeast in an immobilized state as explained below.

Next, a comparison of $\alpha$NE component secreted into the culture medium ($\alpha$NE or Met-$\alpha$NE) treated with CNBr with one which has not been treated with CNBr is carried out by radioimmunoassary using an $\alpha$NE antibody. As seen from the results shown in Table 2, the $\alpha$NE amounts determined in both cases are nearly equal with respect to yeasts transformed by any of the above-explained plasmid. This indicates that

the Met-αNE pre-proprotein produced in the cells is subjected to processing during secretion and mature Met-αNE peptide is secreted in the medium.

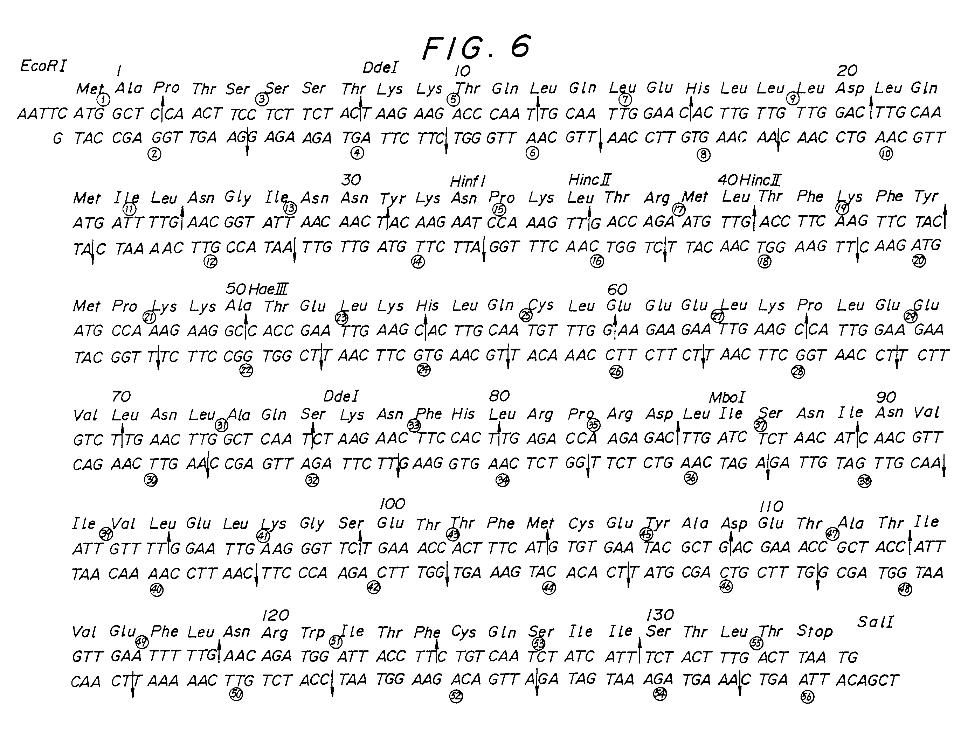

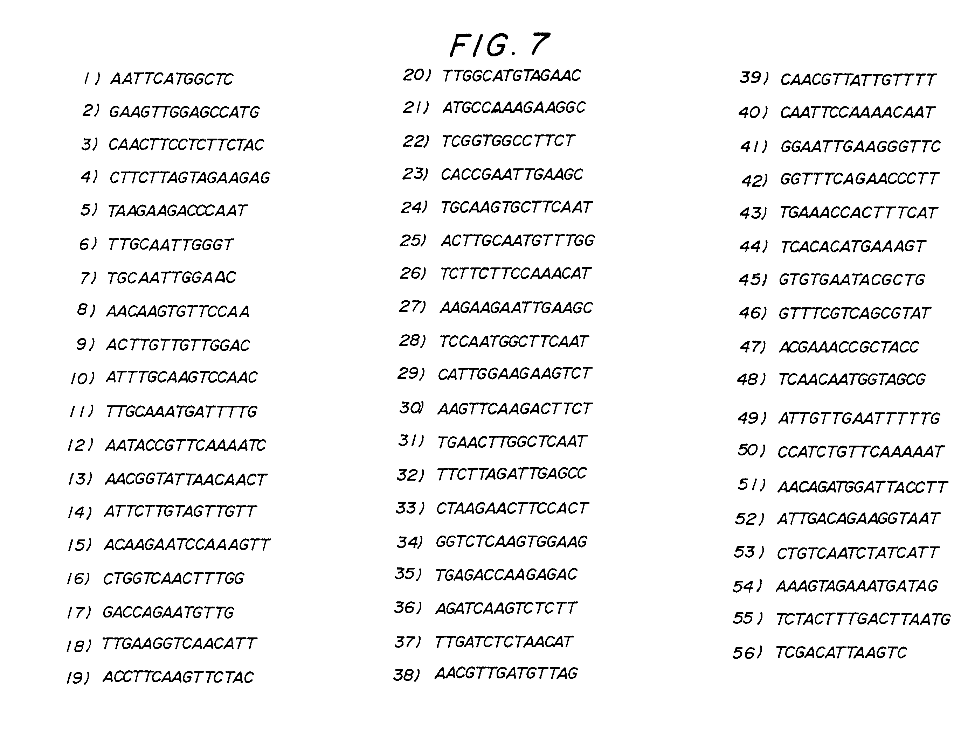

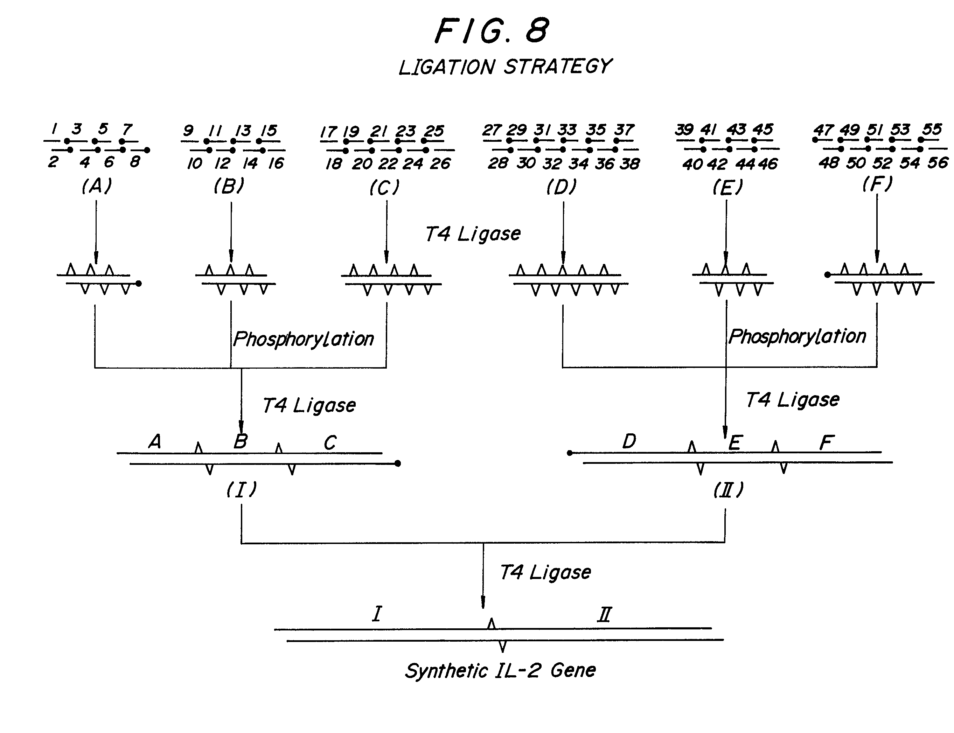

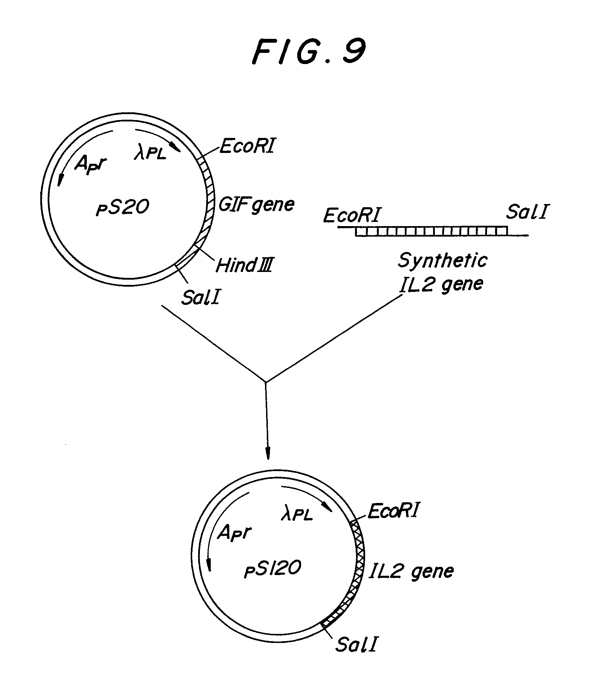

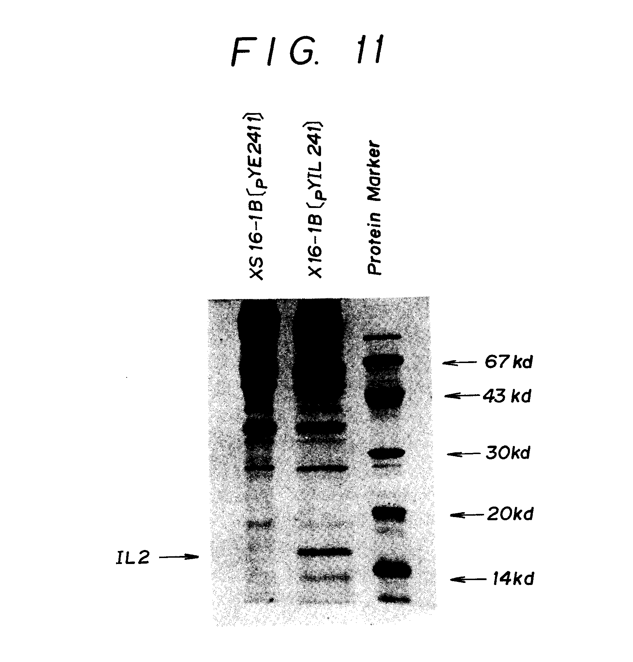

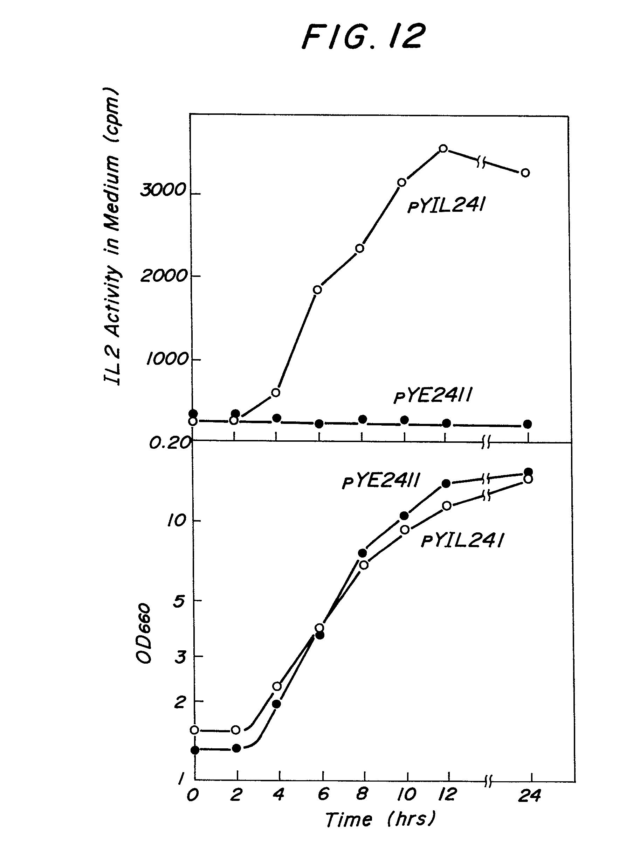

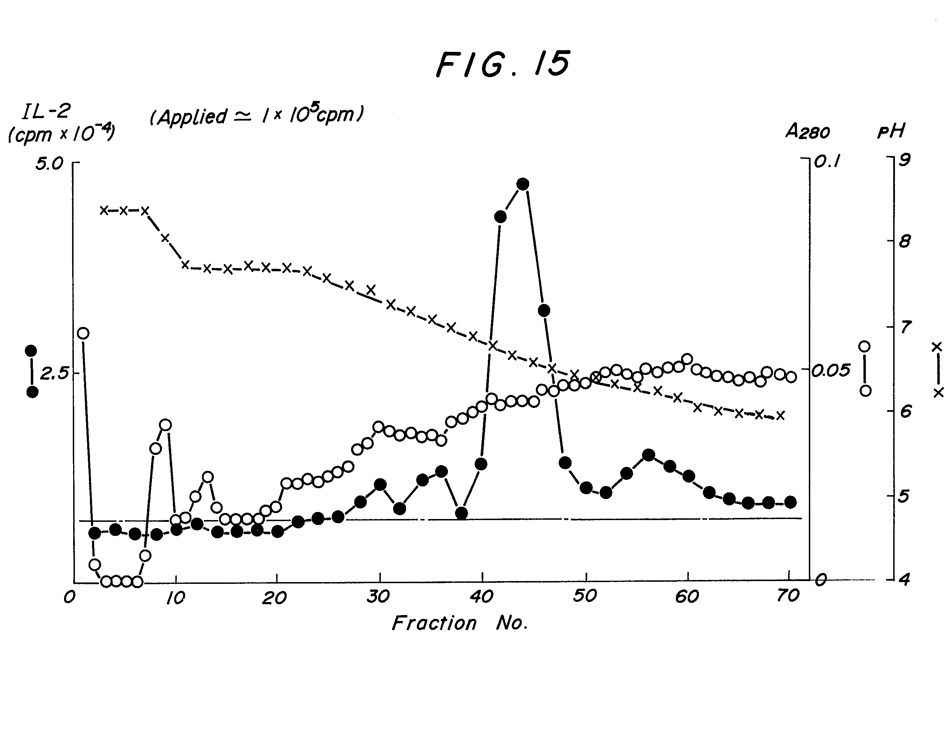

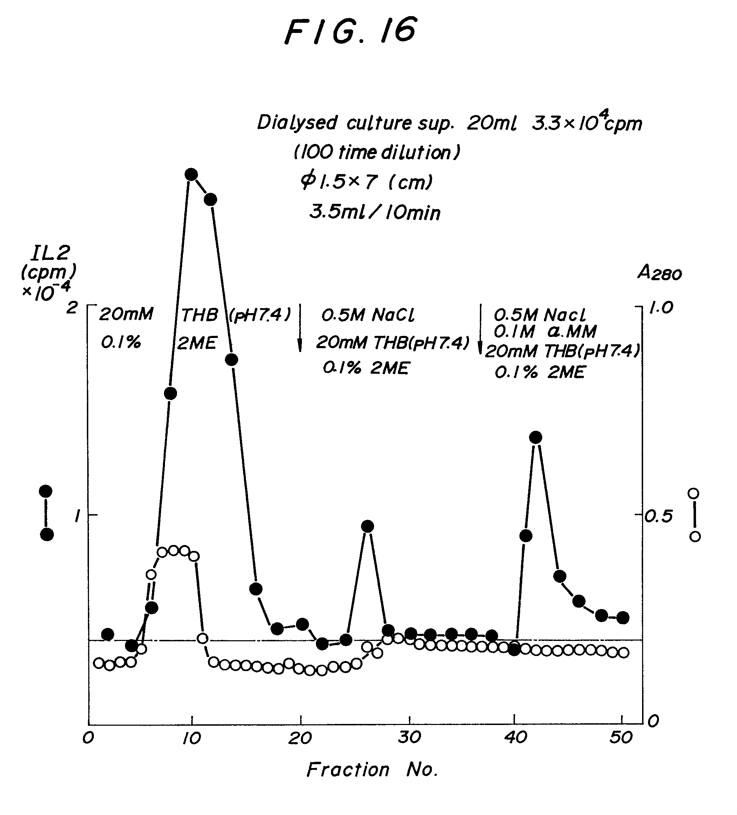

The DNA sequence corresponding to the gene coding for the matured human interleukin 2 (IL2) having a molecular weight higher than that of αNE was chemically synthesized (see Figs. 7 and 8) and used for experiments in the same manner as for αNE. The chemically sunthesized DNA sequence coding for IL2 is shown in Fig. 6 and is called SUN-IL2. The process for constructing a vector pYIL241 for secretively expressing IL2 is shown in Figs. 9 and 11, but the details will be explained by Examples hereunder. In the same manner as in the case of αNE, IL2 is secreted in the culture medium and thus the secretion was found to be a proliferation dependent type (see Fig. 12). Furthermore, as shown in Figs. 15 to 18, separation and purification of IL2 from the culture solution described above and investigation of the physicochemical properties were carried out. What is interesting here is that the fraction having the IL2 activity which is adsorbed to ConA Sepharose and is eluted with α-methyl mannoside was obtained from the components secreted into the medium, as shown in Fig. 16. This strongly suggests that the yeast transformed with pYIL241 secretes in the culture medium IL2 to which saccharide was added.

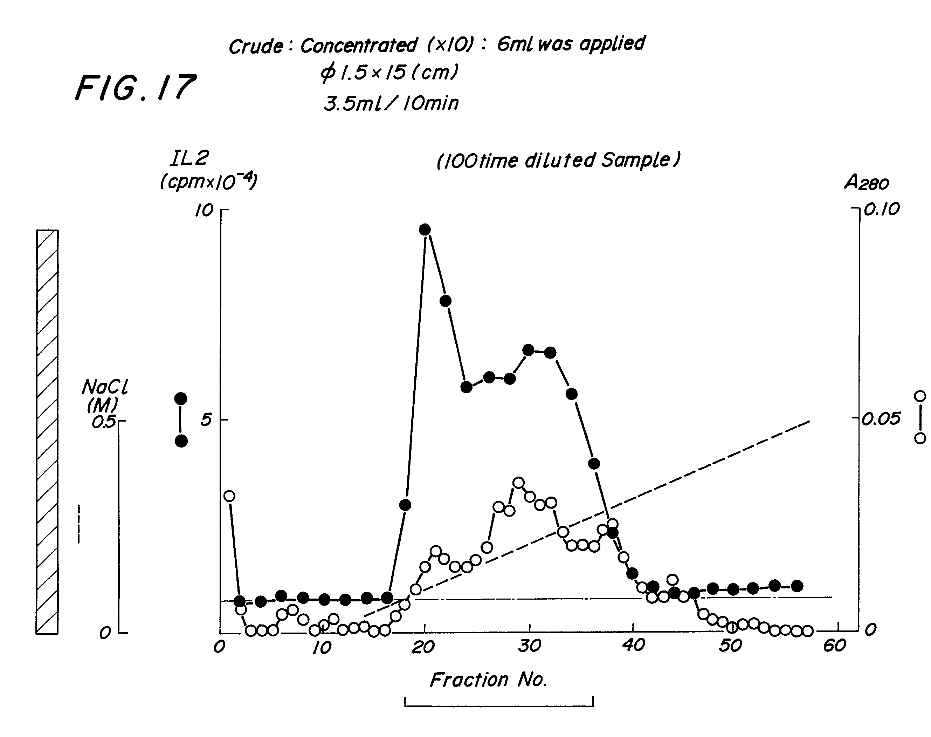

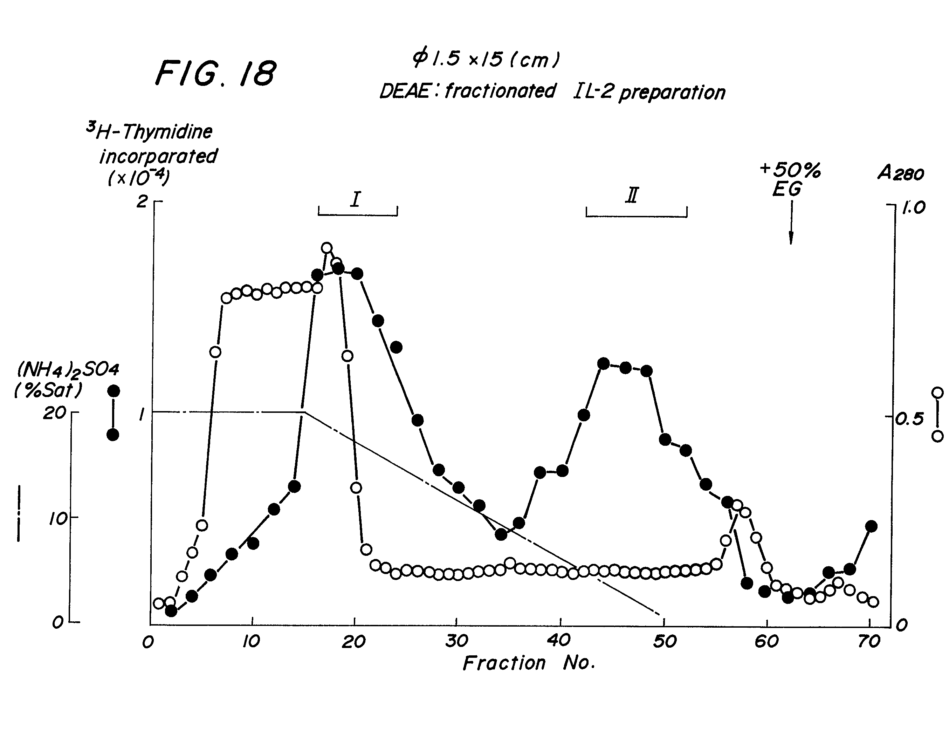

Furthermore, by the chromatofocusing shown in Fig. 15, the IL2 activity was detected to have almost a single peak at a pH around 6.7, but by the ion exchange column chromatography (DEAE-Toyopearl) shown in Fig. 17, the IL2 activity was detected as being a broad peak exhibiting two maximums. By a hydrophobic chromatography of the IL2 active fraction obtained by the above-described DEAE-Toyopearl column chromatography, the active fraction showed the same property of having two maximums as is shown in Fig. 18.

The yeast transformed by the secretor expression vector of the present invention is useful for producing the desired substance on an industrial scale, when a continuous culture is carried out by the immobilized-yeast method which is used for, e.g., ethanol fermentation with a yeast. In particular, it is suitable for a continuous immobilized yeast culture when the MFα promoter is used because the expression of the desired substance is recognized in the logarithmic growth phase. Of course, it is also possible to carry out the continuous culture of immobilized yeast by the use of the secretive expression system of this invention, even if a suitable promoter other than the MFα promoter is used. In case where a promoter of an inducible expression type of gene such as repressible yeast acid phosphatase (PH05) gene is used, it is possible to culture the transformed yeast under non inducing conditions (for PH05, in a medium having a high concentration of phosphate), immobilize the cultured cells, and transfer the immobilized cells under inducing conditions (for PH05, in a low phosphate medium) so that the desired matured protein or peptide can be continuously produced in the cultuture medium.

The transformed yeast of the present invention is immobilized by entrapping cells with various carriers of the generally-known substances such as carrageenan, MCM, gelatin, cellulose triacetate, and polycarbonate. The desired substance can be produced by continuous culture by using the immobilized yeast. The case of κ-carrageenan which is simply referred to as carrageenan hereunder will be explained below.

The Met-αNE or IL2 secretively-producing strains which were proliferated to about $10^7$ cells per 1 ml are mixed with a 4.0% carrageenan solution at 37 - 39°C so as to adjust the cell concentration of the mixture to 5 x $10^7$ cells per 1 ml. The mixed solution is dropwisely added to a 0.3N KCl aqueous solution to entrap the yeast with carrageenan. The immobilized yeast is added to a minimal medium containing 2% Casamino acid and cultured with shaking at 30°C for 24 hours. Then, the yeast is transferred into the same but fresh medium, and this point is regarded as 0 hour for the purposes of investigating change with the passage of time. The culture is carried out with shaking at 30°C, no medium being added during this stage.

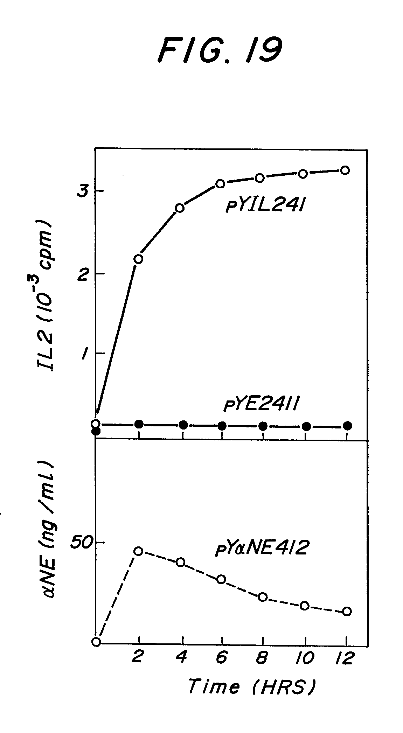

As shown in Fig. 19, the production of IL2 reaches a stationary state at the 6th hour, while it decreases at 2 hours and later in the case of Met-αNE. It can therefore be deduced from the results of the IL2 production that although the secretive expression lasts for 6 hours even with respect to Met-αNE, the decomposition thereof is thought to take place in the culture medium faster than the secretion rate after 2 hours, causing the activity to be decreased.

On the other hand, in IL2 production, IL2 can be produced by a continuous culture in which an exhausted medium is exchanged for the same amount of a fresh one every 6 hours.

Furthermore, it is possible to continuously produce a substance having a low molecular weight such as Met-αNE by incorporating a suitable decomposition inhibitor in the medium.

The present invention will be illustrated on detail by the Example below.

Example

(1) Cloning of MFα genes (Fig. 1)

α-Mating type of haploid Saccharomyces cerevisiae X2180-1B (MATα SUC2 mal gall2 CUPl, available from Yeast Genetic Stock Center, R. Mortimer, University of California, Berkeley, CA 94720) was cultured with shaking in 400 ml of YEPD medium (1% yeast extract, 2% peptone, 2% glucose) at 30°C for 24 hours and the cells were collected by a centrifuge. DNA was separated from the cells and purified according to the method of D. R. Cryer, et al. [Methods in Cell Biology, 12, 39, Academic press (1975)]. The DNA (50 μg) was completely digested with use of 200 units of EcoRI and 200 units of SalI and a DNA fragment of about 1.4 Kb was separated by agar gel electrophoresis. Then the agar gel was cut out and the desired DNA fragment was isolated from the gel by the electroelute method, extracted with phenol, and then precipitated with ethanol. On the other hand, 1 μg of pBR322 was completely digested by using 4 units of EcoRI and 6 units of SalI and a DNA fragment of 3.7 Kb was recovered by the method described before. Then both of these fragments were mixed, dissolved in 20 μl of a ligation

reaction solution (20 mM Tris-HCl pH 7.5, 10 mM $MgCl_2$, 10 mM DTT, 1 mM ATP), and reacted with 2 units of T4DNA ligase at 15°C for 16 hours. To 10 µl of the reaction solution was added Escherichia coli JA221 [cultured to $OD_{660}$ = 0.5 in LB medium (1% trypton, 0.5% yeast extract 0.5% NaCl, pH 7.5)] suspended in 0.3 ml of a 50 mM $CaCl_2$ solution and allowed to stand at 0°C for 40 minutes for transformation. Then, 2.5 ml of the LB medium was added and cultured with shaking at 37°C for 1.5 hours and the obtained solution was spread on a neutrient agar plate containing 50 µg/ml of ampicillin to culture the transformant at 37°C overnight. The plasmid DNA was separated from the cultured ampicillin-resistant transformant by alkali extraction according to the conventional method and analyzed with use of restriction enzymes. A clone containing a plasmid which can be digested with EcoRI and Hind III to give a fragment (ca. 1.3 Kb) and has at least one PstI cutting site was obtained in a proportion of 2 clones out of 300 ampicillin-resistant transformants. The plasmid obtained from one clone from amongst them was cleaved with SalI, subjected to BAP (alkaline phosphatase derived from E. coli) treatment to remove phosphoric acid at 5'-terminal, and determined its DNA sequence labelling the plasmid with $^{32}$P at 5'-terminal and using [γ-$^{32}$P]ATP and T4 polynucleotidekinase by the Maxam-Gilbert method [Meth. Enzym., 65, 499 - 560 (1980)]. The results showed that the sequence was in conformity with that of the MFα gene separated by Kurjan and Herskowitz [Cell, 30, 933 - 943 (1982)]. The plasmid was named as pYMF88. The plasmid was called SBM151 and deposited with Fermentation Research Institute, Japan (International Deposition Acceptance No. FERM BP-382 under Budapest Treaty).

(2) Construction of α-NE secretor expression vector
    (Fig. 2, Fig. 3 and Fig. 4)

A plasmid pYE2411 in which a EcoRI-SalI DNA fragment of pYMF88 (containing the promoter region and the structural gene of MFα) as described in (1) above was inserted in a yeast-Escherichia coli shuttle vector was constructed by the method described below. A DNA fragment of 2.2 Kb containing

a 2 μmDNA replication which was obtained by cleaving 2 μmDNA with EcoRI was joined with a DNA fragment of 0.85 Kb obtained by digesting YRp7 [Struhl, K., etc., PNAS, 76, 1035 - 1039 (1979)] with EcoRI and BglII and with a 4.0 Kb fragment obtained by digesting pBR322 with EcoRI and BamHI to construct a plasmid pYE2001. This plasmid had two EcoRI cutting sites, and, after cleaving the plasmid with EcoRI, both cohesive ends were filled-in by using T4DNA polymelase and 4dNTP and were then ligated with T4DNA ligase to eliminate the EcoRI cutting site because of the inconvenience which would otherwise be involved with the subsequent plasmid construction. Thus, a plasmid pTE2001' without having EcoRI cutting site was constructed. Then, in order to add a terminator region of GAP-DH gene to the plasmid, the GAP-DH gene was obtained by cleaving pYGAP87 (see Japanese Patent Public Disclosure No. 74986/1984) by Hind III, and this was joined to a fragment which had been obtained by partially cleaving pYE2001' with HindIII to construct pYE2201. The SalI cutting site near the TRP1 gene of pYE2201 was then eliminated in the same manner as before to give pYE2202.

The plasmid pYE2202 was partially cleaved with Hind III and joined again through an EcoRI linker so that pYE2203 in which the EcoRI cutting site was formed at a site immediately upstream of the GAP-DH gene was obtained. The MFα gene was obtained by cleaving pYMF88 constructed in Item 1 with EcoRI and SalI and was joined to a DNA fragment which had been obtained by decomposing pYE2203 with EcoRI and SalI thereby constructing pYE2411. The plasmid pYE2411 has a structure in which $_{pre}$MFα is transcribed by the function of the promoter of MF and completed by the terminator of the GAP-DH gene. The plasmid is a shuttle vector which contains a replicon of pBR322 and is selected by β-lactamase gene in an Escherichia coli host, and which contains a 2 μm DNA replicon and is selected by TRP1 gene in a yeast host. The <u>Escherichia</u> <u>coli</u> JA221 transformed by pYE2411 was called SMB275 and deposited with Fermentation Research Institute, Japan (deposition number: FERM P-7728, International Deposition Acceptance No. FERM BP-836).

(3) Construction of αNE expressing vector

The construction of an expression vector of αNE which is a low-molecular weight peptide will be explained below. An αNE gene added a codon (ATG) corresponding to methionine was used so as to make cleavage of the produced protein with CNBr possible. The base sequence is shown in Fig. 3. The upper stream region of αNE gene has a Hind III cohesive end and is designed such that the reading frames are matched when it is joined to HindIII cutting site in MFα NE gene. Furthermore, when it is joined to any of four HindIII cutting sites located immediately upper stream of the DNA sequence coding for MFα which are present in the MFα gene, the reading frames can be matched with each other. This αNE gene was obtained by chemically synthesizing 8 DNA fragments (F1 - F8) as shown in Fig. 3 by the method described in Japanese Patent Public Disclosure No. 63395/1983, joining these fragments with T4DNA ligase, and then separating and purifying the resulting αNE gene by using a polyacrylamide gel. Since the BamHI cohesive end sequence is located at the downstream of the αNE gene, the SalI cutting site of the vector pYE2411 to be inserted was changed into the BamHI cutting site to construct pYE2411-BamHI (Fig. 4). That is to say, 5 µg of pYE2411 was cleaved by using 20 units of SalI, and after filling in the SalI cohesive end by using T4DNA polymelase and 4dNTP, the BamHI linker was added to construct pYE2411-BamHI by the method previously described. On the other hand, 15 µg of pYMF88 was completely cleaved by using 60 units of EcoRI and then partially cleaved at 37°C for 15 minutes by using 1 unit of Hind III. The reaction solution was separated by agar gel electrophresis and thereby a DNA fragments corresponding to about 1.3 Kb to about 1.5 Kb were obtained by the method previously described. They were mixed with 0.1 µg of a ligase reaction solution and reacted by using 5 units of T4DNA ligase at 15°C for 16 hours. The Escherichia coli JA221 was transformed by using 10 µl of the ligated substance to obtain an ampicillin resistance clone. The analysis was carried out by using several restriction enzymes in accordance with the conventional method. The results showed that clones containing pYαNE412, pYαNE413, pYαNE414 and pYαNE415 were produced (Fig. 4).

(4) Secretor expression of pYαNE412, pYαNE413, pYαNE414 and pYαNE415

The plasmid obtained in Item 3 was used to transform haploid Saccharomyces cerevisiae XS16-1B (MATα leu2 his3 trpl) in accordance with the general method to obtain TRP$^+$ clone. A TRP$^+$ clone transformed with pYE2411 was also obtained to be used for comparison. The transformants obtained were cultured with shaking in the CA medium (0.6% Difco Nitrogen base w/o amino acid, 2% glucose, 2% Casamino acid) at 30°C. The results obtained from the assay of the αNE activity are shown in Table 1, with respect to the 9.5-hour (logarithmic growth phase) and the 24-hour (stationary phase) cultures. This assay was carried out by RIA using an antibody against αNE in accordance with the method described in Japanese Patent Public Disclosure No. 63395/ 1983. The cells and the culture supernatant were separated by centrifuging 1 ml of the culture solution sampled at each phase. at 10,000 rpm for 5 minutes. The cells were treated with CNBr in accordance with the method described in Japanese Patent Public Disclosure No. 63395/1983, and used as a sample. The culture supernatant liquid per se was used as a sample. Table 1 shows the αNE activity in terms of 1 ml of the culture liquid. No αNE activity was detected in the transformant with pYE2411 which was constructed for comparison. The cell proliferation corresponded to $OD_{660} \fallingdotseq 3.5$ in 9.5 hours and $OD_{660} \fallingdotseq 15$ in 24 hours. The secretion ratio (% Secretion) in Table 1 was calculated by dividing the value of the culture supernatant liquid activity by the sum of the cell activity and the culture supernatant liquid activity. The sample at 9.5 hour cultivation exhibited a secretion ratio of 19 - 38%. The secretion ratio was decreased to 3.3 - 11% on 24 hour cultivation, but the cellular activity per cell (per $OD_{660}$) was essentially the same as 9.5 hours. Therefore, although the secretor expression was recognized even if a gene coding for a desired protein or peptide is inserted at any of four sites each of which is the starting site of a DNA sequence coding for matured MFα, the insertion at an upstream site showed a tendency to increase the secretion efficiency.

A comparison with the sample treated with CNBr was then carried out with respect to the activity of the culture supernatant. The RIA of the sample which was treated with CNBr by dissolving in a 70% formic acid solution containing 5 mg/ml of CNBr after freeze-drying the supernatant liquid of the sample incubated for 24 hours was compared with that of the sample not treated with CNBr. The results are shown in Table 2. From the results the reactivities with the α NE antibody showed nearly the same values. It was found from these results that processings during secretor expressing were correctly made.

The change with-passing of time was investigated by using the transformed yeast XS16-1B (pYα NE412). The shaking culture was carried out at 30°C by using the CA medium. The results are shown in Table 5. The cell proliferation is represented by the value of $OD_{660}$ and the αNE activity is shown as the result from RIA by the same method as before. Although the intracellular α NE value increases in close correspondence with proliferation of cells (that is to say, the α NE value per cell was the same), the activity in the culture supernatant did not show a remarkable increase. The intracellular activity level was also decreased with 24 hour cultivation. These results show that the α NE was decomposed in the medium. It is however believed that the problem can be solved by using a suitable decomposition inhibitor in the culture medium.

The secretor expression using the MFα gene system was investigated with respect to a protein having a high molecular weight by using IL2 which is an immune-related substance.

(5) Chemical synthesis of IL2 gene (Fig. 6 to Fig. 8)

The human IL2 is a polypeptide consisting of 133 amino acid residues and its amino acid sequence was determined by gene analysis [Taniguchi, et al., Nature, 302, 305 - 310 (1983)].

1)    An optimal codon of a yeast was selected as the codon corresponding to each amino acid as much as practicable.

2)    Met was coded immediately before Ala at N-terminal of IL2 and a terminating codon (TAA) was introduced immediately after Thr of the C-terminal.

3)    The EcoRI cohesive end was added to the +strand 5'-terminal of the synthesized gene and the SalI cohesive end was added to the -strand 5'-terminal in order to make easier to insert the synthetic IL2 gene into a vector.

The whole gene was divided into 56 kinds of oligonucleotide fragments (12Nt - 17Nt) as shown in Fig. 7 and 8 in order to synthesize the IL2 gene designed in the above-mentioned manner.  The method of division was designed so that the complementary strands overlapped by at least 5 bp.

The 56 kinds of DNA fragments shown in Fig. 8 were chemically synthesized by using the solid-phase triester method.  The ligation strategy for constructing the final IL2 gene from the 56 kinds of chemically synthesized fragments by the T4DNA ligase reaction will be further illustrated in the following processes 1 to 3.

Process 1 : Synthesis of A to F blocks

As shown in Fig. 8, 1 nmol of each one of 46 fragments in which fragments 1 and 56 were excluded from A to F blocks and in which the fragments at the 5'-termial of each block (9, 16, 17, 26, 27, 38, 39, 46) were excluded from B block to E block was reacted with 8 Ci units of $[\gamma-32_P]$ATP (2800 Ci/mmol), 5 units of polynucleotidekinase, 70 mM Tris-HCl (pH 7.5), and 30 μl of a 10 mM $MgCl_2$ solution at 37°C for 30 minutes to label the 5'-terminal with $^{32}P$. Consequently, 8 nmol of ATP and 5 units of polynucleotidekinase were added, and the resulted solutions were reacted at 37°C for 60 minutes and heated at 90°C for 5 minutes. Each of the fragments (0.6 nmol) which was not phospholylated was mixed with a half amount of the above reaction solutions separately for each of A to F blocks, dialyzed against distilled water at 4°C for 16 hours, and then concentrated to dryness.  Then, the obtained substances were dissolved in 5 µl of the ligase buffer solution (40 mM Tris-HCl pH 7.5, 20 mM $MgCl_2$) and then sealed in a 40 µl capillary. The capillary was put in a 16.5 mm test tube and the test tube was placed in a beaker for heating at 90°C for 2 minutes. Then the beaker was allowed to stand at room temperature for 1.7 hours to lower the temperature to 37°C, then to stand at 37°C for 40 minutes, and finally the temperature was lowered to 20°C over a period of 40 minutes. Thereafter, the beaker was allowed to stand at 8°C for 60 minutes for annealing the DNA fragments. After the DNA solutions were taken out, 2.0 mM MATP 10 mM DTT and 15 units of T4DNA ligase were added, and reacted at 8°C for 5 hours in the resulting solution in a total volume of 38 µl. When a portion of the solution was analysed by 14% polyacrylamide electrophoresis containing 7M urea 1 hour after completion of the reaction, a sufficient amount of the intended substance was found to have been produced. The all reaction solution was, in a similar manner, separated by 14% polyacrylamide electrophoresis containing 7M urea. The gel of the intended portion was cut out, homogenized and then extracted with 0.5M ammonium acetate, 0.01M magnesium acetate, 0.1% sodium dodecyl sulfate, and 0.1 mM EDTA at 65°C for 16 hours. DNA of A - F blocks was then recovered from the solution by ethanol precipitation.

Process 2 : Synthesis of I and II blocks

200 pmol of B, C, D and E blocks were, in a manner similar to Process 1, reacted with 2.5 units of polyneceotidekinase, 9 µCi units of $[\gamma-^{32}P]$ ATP (5200 Ci/mmol) and then with 8 nmol of ATP to phospholylate the 5'-terminals in a solution in a total volume of 15 µl. The solution was mixed with 200 pmol of A and F blocks, respectively, and 5 units of 10 mM DTT, T4DNA ligase were then added and reacted at 20°C for 5 hours in a total colume of 50 µl. As in the case of Process 1, a portion of the reaction solution was analysed by 8% polyacrylamide electrophoresis containing 7M urea, the intended substance was found to be produced in a good yield. Therefore, the total volume of the solution was separated by 8% polyacrylamide electrophoresis to obtain blocks I and II.

Process 3

Blocks I and II (each 100 pmol) were dissolved in 45 µl of a solution of 0.4 mM ATP, 10 mM DTT, 20 mM Tris-HCl pH 7.5, and 10 mM MgCl$_2$, 5 units of T4DNA ligase were added and the resulting solution was then reacted at 37°C for 1.5 hours in a solution in a total volume of 50 µl. A portion of the solution was analysed by 8% polyacrylamide electrophoresis containing 7M urea, and the intended substance was found to have been produced. Therefore, the total volume of the solution was separated by 5% polyacrylamide electrophoresis containing 7M urea. In a similar manner, the intended substance was eluted from the gel, desalted, and then reacted with 8 nmol ATP and 5 units of polynucleotidekinase at 37°C for 60 minutes in a total colume of 10 µl to obtain IL2 DNA for transformation. The IL2 DNA sequence is shown in Fig. 6 and is called SUN-IL2.

(6) Expression of chemically synthesized IL2 gene in Escherichia coli (Fig. 9)

Two µg of the plasmid vector pS20 containing the λPL promoter, the SD region of a gene for λCII protein and γ-IFv gene [described in Japanese Patent Public Disclosure No. 116605/1984 and Escherichia coli transformed by this vector (N4830/pS20) was called SBM271 and deposited with Fermentation Research Institute (International Deposition Acceptance No. FERM BP-535)] was completely cleaved by using 10 units of EcoRI and SalI to obtain a DNA fragment. of about 4.2 Kb excluding the γIFN genes by the method described in Item 1. The DNA fragment and 0.1 µg of the chemically synthetic IL2 gene were d゛゛solved in 20 µl of a ligation reaction solution (described before) and subjected to a reaction by using 2 units of T4DNA ligase at 15°C for 18 hours. The reaction solution (10µl) was used to transform Escherichia coli N4830 (described in Japanese Patent Public Disclosure No. 116605/1984) by the method described before. The obtained ampicillin-resistant clone was analyzed by the conventional method. The results showed that a clone containing pS120 which was the plasmid with the IL2 gene inserted in the place of γ-IFN gene of pS20 had been produced (Fig. 9). The productivity of substances having IL2 activity was measrued in respect to 10 of the obtained clones. The method of IL2 activity measurement will be explained later. An induction was carried out by raising the temperature of the culture solution to 42°C after cultivation in the LB medium (described before) to $OD_{660} \doteqdot 0.5$ at 32°C. After induced at 42°C for 45 minutes 1 ml of the culture solution was centrifuged (10,000 rpm, for 5 minutes) to recover the cells. The cells were suspended in 1 ml of PBS solution containing 1 mg/ml of lysozyme (obtained from Nissui Seiyaku Co., Ltd.) and allowed to stand at 4°C for 30 minutes. The cells were disrupted by repeating a freeze/thaw process 3 times with an ethanol-dry ice bath and a 30°C water bath. The IL2 activity of the supernatant centrifuged at 10,000 rpm for 5 minutes was investigated by measuring the uptake of $^3$H-thymidine of the IL2-depending cells (as described later, using human HL-30221). All of the 10 clones containing pS120 showed the IL2 activity. A solution extracted from the cells of the same clones which had not been exposed to a temperature of 42°C showed no IL2 activity. The results using one clone of cells are shown in Table 3. For the purposes of comparison, the results obtained using host cells and no induced cells are also shown as controls in Table 3. Subsequently, an analysis by SDS polyacrylamide gel electrophoresis (SDS-PAGE) was carried out. N4830/pS120 was cultured at 32°C in M9AA which was prepared by adding 19 amino acids excluding methionine to M9 minimal medium for a time sufficient to reach $OD_{660} \doteqdot 0.4$, and then the temperature was raised to 42°C. After 15 minutes or 30 minutes, 10 μCi of $^{35}$S-methionine per 0.3 ml culture solution was added and allowed to stand for 2 minutes to complete labelling and then TCA was added to the solution so as to become a final concentration to 10% followed by allowing the solution to stand on ice for 10 minutes. The precipitates were recovered by centrifuging at 10,000 rpm for 5 minutes. The precipirates were isolated by SDS-15% polyacrylamide gel electrophoresis (SDS-PAGE) according to the conventional method and the expressed protein was detected by subjecting the gel to radio-autography. As a result, a band of about 16 Kb which appeared by the induction at 42°C was recognized. It was found from these results that IL2 gene obtained by chemical synthesis expressed IL2 protein.

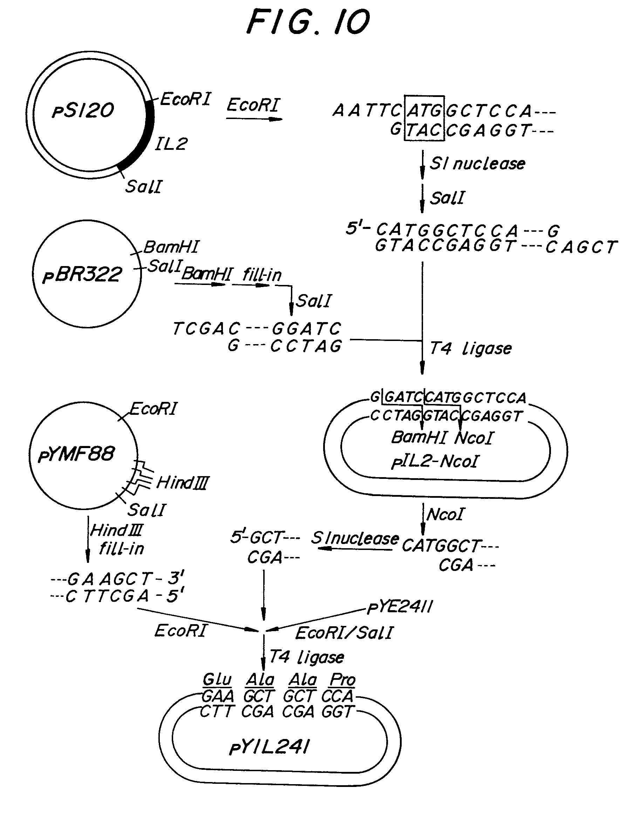

(7) Introduction of IL2 gene into yeast secretory vector (Fig. 10)

A initiation codon is added to the IL2 gene constructed in Item 5 in order to make direct expression possible. In addition, the IL2 gene cannot be directly jointed to the MFα gene because the joining site is an EcoRI cutting site. Therefore, an IL2 expression vector pYIL241 utilizing the MFα gene system was constructed by the method described below. The plasmid pS120 (10 μg) was digested by using 40 units of EcoRI and then DNA was recovered by ethanol precipitation. The DNA was dissolved in 100 μl of a S1 nuclease buffer solution (28 mM NaCl, 50 mM sodium acetate, 4.5 mM $ZnSO_4$, pH 4.6) and reacted with 2 units of S1 nuclease (produced by PL) at 30°C for 15 minutes. After completion of the reaction, an extraction was carried out with phenol once and the DNA was recovered by ethanol precipitation.

By the complete digestion using 60 units of SalI, the DNA fragment containing IL2 gene of about 0.4 Kb was obtained by the method described above. On the other hand, 3 μg of pBR322 was cleaved using 15 units of BamHI, filled-in the cohesive ends by using T4DNA polymelase and 4dNTP, and then digested by using 15 units of SalI to obtain the DNA fragment of 4.1 Kb by the method previously described. Both of the fragments were mixed, dissolved in 20 μl of a ligation reaction solution, and reacted using 5 units of T4DNA ligase at 15°C for 18 hours. Escherichia coli JA221 was transformed by using 10 μl of the reaction solution. The obtained ampicillin resisting clone was analyzed according to the general method, and it was found that the clone containing plasmid pIL2-NcoI had been obtained. The plasmid pIL2-NcoI (10 μg) was then completely digested with 40 units of NcoI and then subjected to a SI nuclease reaction to remove the cohesive ends by the method previously described. By the complete digestion using 60 units of SalI, the DNA fragment containing IL2 gene of about 0.4 Kb was obtained by the method previously described. Separately, pYMF88 (5 µg) was digested using 20 units of HindIII, and after filling-in the cohesive ends by usign T4DNA polymelase and 4dNTP, completely digested with 20 units of EcoRI to obtain a DNA fragment of about 1.3 Kb containing the MFα promoter in accordance with the method previously described. On the other hand, 5 µg of pYE2411 was completely digested using 20 units of EcoRI and 30 units of SalI to obtain thereafter the DNA fragment of about 7.1 Kb not containing the MFα gene. The obtained 3 kinds of DNA fragments were mixed and reacted in the presence of 5 units of T4DNA ligase at 15°C for 18 hours. Escherichia coli JA221 was transformed by using 10 µl of the reaction solution.

The analysis of the obtained ampicillin-resitant clone by the conventional method showed that a clone which is believed to contain pYIL241 had been obtained.

(8) Secretor expression in yeast

Six clones which seemed to contain pYIL241 were selected from those obtianed in Item 7, and the plasmid DNA was separated and purified in accordance with the conventional method. They were used to transform α-mating type of haploid Saccharomyces cereviaiae XS16-1B by the method previously described. The obtained TRP$^+$ transformant was cultured with shaking in the CA medium (described above) at 30°C. After culturing for 16 hours, IL2 activity was measured with respect to the culture supernatant which was obtained by centrifugating at 10,000 rpm for 5 minutes (Table 4, using human HL-30221). As a result, the activity was confirmed in the culture supernatant of the transformant obtained by using the plasmid pYIL241 DNA separated from 3 clones out of 6 clones. After concentration of the culture supernatant by using Centricon-10 (produced by Amicon Co., Ltd.), the sample was subjected to SDS-PAGE. The results are shown in Fig. 11. A band originated from pYIL241, which does not appear with respect to the host, was found at the position corresponding to about 16 Kb. It is concluded from this result that the IL2 protein was secretively produced in an amount of about 0.2 μg in terms of 1 ml of the culture supernatant. Then, the change of the IL2 secretor expression with time passage was investigated. The yeast transformed with pYE2411 was used for a parallel running as control. The cultivation was carried out according to the method previously described (in Item 4) and IL2 activity was measured with respect to the culture supernatant obtained by centrifugating at 10,000 rpm for 5 minutes. The results are shown in Fig. 12. The secretor expression of IL2 was recognized in parallel with proliferation of the transformant. That is to say, the increase in IL2 activity in the medium was recognized in the growth phase over 12 horus, as seen in Fig. 12. Furthermore, no IL2 expression was recognized with respect to the a- mating type of haploid yeast transformed with pYIL241 and, therefore, this indicates that the secretor expression depends on the MFα promoter.

(9) Measurement of IL2 activity

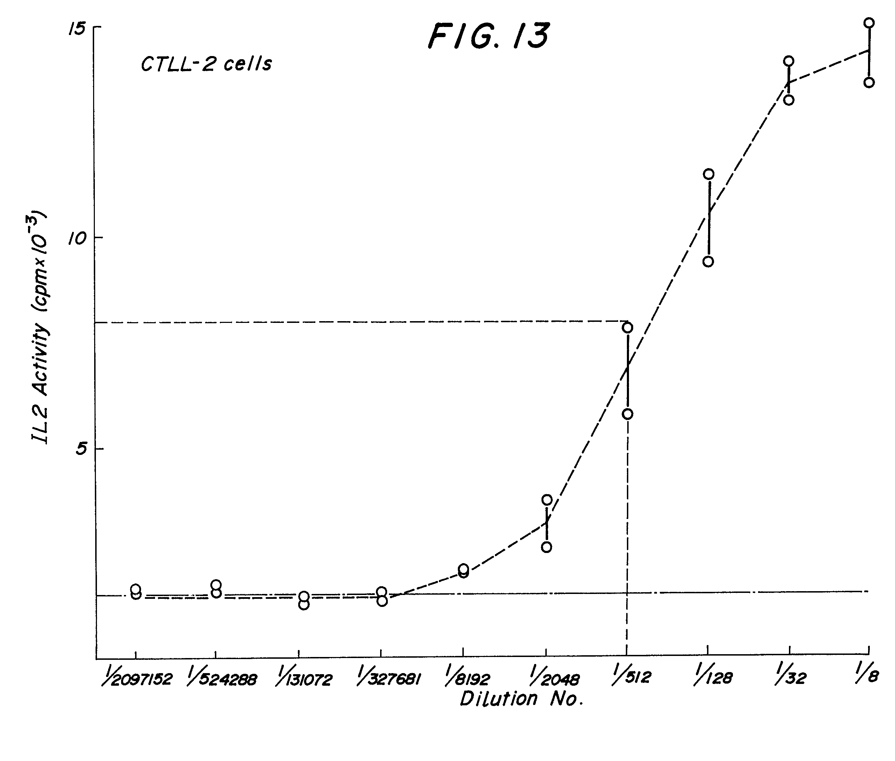

The IL2 activity was measured in terms of the amount of uptake of tritium thymidine with respect to mouse CTIL-2 [Baker, et al., J. Exp. Med., 149, 273 (1979)] and human HL-30221 (distributed by Prof. Omaeda of Kyoto University) which are IL2 dependent cell lines. The cultures supernatant (called crude IL2 below) of the yeast in the CA medium was diluted with RPMI-1640 medium (containing 2.5% fetal bovine serum and $5 \times 10^{-5}$M 2-mercaptoethanol in the case of CTIL-2, or containing 20% fetal bovine serum in the case of HL-30221 cell) to form a 4 time dilution series on a microtiter plate of 96 wells. $2 \times 10^4$ cells were added to each of the wells to produce a final liquid volume of 200 μl. After a 20-hour (CTIL-2 cell) or 44-hour (HL-30221 cell) culture in a $CO_2$ incubator (5% $CO_2$, at 37°C), 0.25 μCi (8 Ci/mmole) of tritium thymidine was added to each of the wells and allowed to culture for further 4 hours, and then the cells were collected on a glass fiber filter, and washed thoroughly with water. Then, the amounts of tritium taken up in the DNA were measured by a liquid scintillation counter (Packed TRI-CARB-460C). Fig. 13 shows the increase in uptake of tritium which depends upon the concentration of crude IL2. IL2 activity showing 50% of the maximum amount of uptake was regarded as 1 unit.

(10) Effect of crude IL2 on CTIL-2 cell proliferation

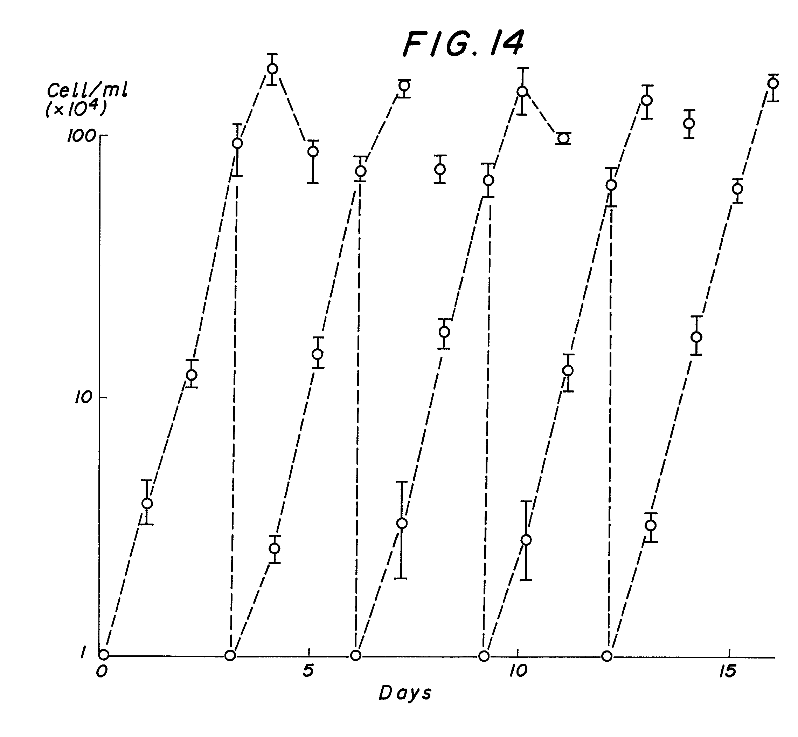

The CTIL-2 cells were subcultured in RPMI-1640 medium (containing 10% fetal bovine serum and $5 \times 10^{-5}$ M 2-mercapto-ethanol) containing 10% (v/v) crude IL2 (Fig. 14). The CTIL-2 cells in the medium containing the crude IL2 proliferated by about 4 times every day, but the proliferation did not take place in the medium free from the crude IL2, causing almost all cells to die 2 days from removal of IL2. Furthermore, no IL2 activity was recognized for the culture supernatant from the cultivation for 3 days.

(11) Biochemical property of yeast IL2

(1)     Fractionation of crude IL2 by chromatofocusing
        (Fig. 15)

The crude IL2 (100 ml) was concentrated by Centricon-10 (Amico Co., Ltd.) to 10 ml, dialyzed against 0.025 M tris-HCl buffer solution (pH 8.0) and then fractionated by using a poly-buffer and an ion-exchange resin PBE94 column (Pharmacia Co., Ltd. 1.5 x 15 cm) which had been equilibrated with the same buffer. IL2 was eluted with 250 ml of the 10-fold diluted poly-buffer (Pharmacia Co., Ltd.) (poly-buffer 74:96 = 7:3, final pH 5.0), and the IL2 activity was eluted as a single peak near pH 6.7, as shown in Fig. 15.

(2)     Fractionation of crude IL2 by Con-A Sepharose
        (Fig. 16)

The crude IL2 (10 ml) was fractionated by a Con-A Sepharose 6B (Pharmacia Co., Ltd. 1.5 x 7 cm) equilibrated with 20 mM Tris-HCl buffer solution (pH 7.4) (Fig. 16).

The IL2 activity was recovered in both the unadsorbed fraction and the fraction adsorbed and eluted with 0.1 M $\alpha$-methyl-mannoside (produced by Nakarai Kagaku Co., Ltd.), and proportion of the activity between the fractions was estimated to be 4:1 on the basis of the standard reaction curve. The results indicate that IL2 which had bonding sites in common with $\alpha$-methyl-mannoside to concanavalin A was contained in the crude IL2 fractions in an amount of about 20% of the total IL2 activity.

Mier and Gallo [J. Immuno, 128, 1122 (1982)] and Welte, et al. [J. Exp. Med., 156, 454 (1982)] have reported that IL2 produced by stimulating lymphocyte with phyto-hemagglutinin did not bond to Con-A Sepharose. Thereafter, it can be concluded that the Con-A Sepharose-adsorbed fraction recognized here was of characteristics inherent to the IL2 produced by the present production system.

(3)     Fractionation of crude IL2 by ion exchange column

The crude IL2 (200 ml) was dialyzed against 20 mM Tris-HCl buffer solution (pH 7.4) and subjected to a DEAE-Toyo pearl column (Toyo Soda, 1.5 x 15 cm) which had been equilibrated with the same buffer, and the adsorbed fraction was fractionated by increasing the salt strength with NaCl (Fig. 17). The IL2 activity was eluted as a broad peak having two maximums. In the case of fractionation by CM-Toyopearl under the same conditions, IL2 was fractionated into unadsorbed and adsorbed fractions.

(4)     Fractionation by hydrophobic chromatography

Ammonium sulfate was added to the DEAE-Toyopearl-eluted fraction so as to produce 20% saturation and this was subjected to a buthyl-Toyopearl column (Toyo Soda, 1.5 x 15 cm) and fractionated by decreasing the salt strength. The IL2 activity showed a curve having two peaks, as shown in Fig. 18. It was concluded that this result was caused by the heterogeneous properties of IL2 due to the presence of saccharide chains structure, etc.

(12) Secretor expression by immobilized-yeast

Each of the transformed yeast XS16-1B [pYαNE412] and XS16-1B [pYIL241] obtained in Items 4 and 8 was proliferated in the CA medium until reaching about $10^7$ cells per 1 ml, mixed with 40% carrageenane solution (supported at 37°C - 30°C) so as to become 5 x $10^7$ cells per 1 ml, and then immediately added dropwise to a 0.3 N KCl solution to entrap and immobilize the yeast onto the carrageenane. The immobilized yeast was added to the minimal medium containing 2% Casamino acid and cultured with shaking at 30°C for 24 hours.  The immobilized yeast was transferred into a fresh portion of the same medium and this point was regarded as 0 hour for the purposes of investigating the change with passage of time.  The culture was carried out with shaking at 30°C, no medium being added during the culturing.  The results are shown in Fig. 19.

The production of IL2 reached a stationary stage after 6 hours, but in the case of α NE, it decreased after 2 hours.  From this result, it is concluded that although the secretor expression of α NE lasted in the same way as IL2 over 6 hours, the decomposition rate in the medium took place faster than the expression.

The IL2 production was possible by a continuous culture in which a fresh medium was replaced every 6 hours, and the IL2 secreted was stable in the medium.

On the other hand, peptide of a small molecular weight such as α NE is unstable in the medium, but continous culture is thought to be possible, for example, by adding a decomposition inhibitor.

Table 1 : Production of αNE

| Plasmid | 9.5-hour culture [1] | | | 24-hour culture [1] | | |
|---|---|---|---|---|---|---|
| | αNE in cell | αNE in super-natant liquid [2] | Secretion ratio (%) [3] | αNE in cell | αNE in super-natant liquid [2] | Secretion ratio (%) [3] |
| pYαNE412 | 54 μg/ml | 20 μg/ml | 27% | 200 μg/ml | 26 μg/ml | 11% |
| pYαNE413 | 28 μg/ml | 17 μg/ml | 38% | 232 μg/ml | 22 μg/ml | 9.4% |
| pYαNE414 | 36 μg/ml | 12 μg/ml | 25% | 188 μg/ml | 8 μg/ml | 4.1% |
| pYαNE415 | 63 μg/ml | 15 μg/ml | 19% | 290 μg/ml | 10 μg/ml | 3.3% |

[1]  OD ≒ 3.5 (9.5 hours, OD ≒ 15 (24 hours)

[2]  Culture supernatant without CNBr treatment.

[3]  Secretion ratio = $\dfrac{(\text{αNE in culture supernatant})}{(\text{αNE in cell}) + (\text{αNE in culture supernatant})}$

0171000

-29-

Table 2 : Effect of CNBr treatment on reactivity with
anti-αNE antibody in culture supernatant

| Plasmid | Reactivity of αNE with anti-α NE antibody (ng) | |
| --- | --- | --- |
| | CNBr treatment | without CNBr treatment |
| pYαNE412 | 25.7 | 25.3 |
| pYαNE413 | 30.5 | 32.5 |
| pYαNE414 | 8.3 | 8.0 |
| pYαNE415 | 9.2 | 8.2 |

The yeast transformant obtained by use of each of the
plasmids was cultured at 30°C for 24 hours and the culture
supernatant was obtained by centrifugation.

Table 3 : IL2 activity by N4830/pS120

| Yeast | Uptake of $^{3}$H-thymidine |
| --- | --- |
| N4830/pS120 (induction) | 3071 ± 165 cpm |
| N4830/pS120 (non-induction) | 78 ± 15 cpm |
| N4830 | 80 ± 19 cpm |

Table 4 : IL2 activity in the medium

| Yeast | IL2 activity |
| --- | --- |
| XS16-1B pYIL241 | 5320 ± 1061 cpm |
| XS16-1B pYE2411 | 1159 ± 61 cpm |

Claims:

1. A method of producing a protein or peptide which comprises culturing yeast transformant immobilized onto a carrier gel, and isolating the product from the culture medium, said yeast transformant being transformed by a secretor expression vector which is constructed by replacing a part or whole of a DNA sequence coding for a matured $\alpha$-factor (MF$\alpha$) peptide in an MF$\alpha$ operon of yeast by a DNA sequence coding for the object protein or peptide, and optionally exchanging a promoter of MF$\alpha$ for another promoter derived from yeast.

2. A method according to Claim 1 wherein said object protein is a human or mammal immune-related substance or its analogous substance.

3. A method according to Claim 2 wherein said immune-related substance or its analogous substance is a substance having human interleukin 2 (IL2) activity.

4. A method according to Claim 1 wherein said object protein is one which is glycosilated.

5. A method according to Claim 4 said protein is one having human interleukin 2 (IL2) activity.

6. A method according to Claim 5 wherein said protein is able to be adsorbed on ConA Sepharose and eluted by $\alpha$-methylmamannoside.

7. A method according to Claim 1 wherein said object peptide is a human or mammal peptide hormone or its analogous peptide.

8. A method according to Claim 7 wherein said peptide hormon is $\alpha$-neoendorphin ($\alpha$-NE).

9. A method according to Claim 1 wherein a DNA sequence coding for the object protein or peptide is one which is chemically synthesized.

10. A method according to Claim 9 wherein said chemically synthesized DNA sequence is represented by the following sequence:

```
AGCTATGTATGGCGGTTTCCTTCGTAAGTATCCGAAGTAATAG
    TACATACCGCCAAAGGAAGCATTCATAGGCTTCATTATCCTAG.
```

11. A method according to Claim 9 wherein said chemically synthesized DNA is represented by the following sequence:

```
AATTC ATG GCT CCA ACT TCC TCT TCT ACT AAG AAG ACC
    G TAC CGA GGT TGA AGG AGA AGA TGA TTC TTC TGG

CAA TTG CAA TTG GAA CAC TTG TTG TTG GAC TTG CAA ATG
GTT AAC GTT AAC CTT GTG AAC AAC AAC CTG AAC GTT TAC

ATT TTG AAC GGT ATT AAC AAC TAC AAG AAT CCA AAG TTG
TAA AAC TTG CCA TAA TTG TTG ATG TTC TTA GGT TTC AAC

ACC AGA ATG TTG ACC TTC AAG TTC TAC ATG CCA AAG AAG
TGG TCT TAC AAC TGG AAG TTC AAG ATG TAC GGT TTC TTC

GCC ACC GAA TTG AAG CAC TTG CAA TGT TTG GAA GAA GAA
CGG TGG CTT AAC TTC GTG AAC GTT ACA AAC CTT CTT CTT

TTG AAG CCA TTG GAA GAA GTC TTG AAC TTG GCT CAA TCT
AAC TTC GGT AAC CTT CTT CAG AAC TTG AAC CGA GTT AGA

AAG AAC TTC CAC TTG AGA CCA AGA GAC TTG ATC TCT AAC
TTC TTG AAG GTG AAC TCT GGT TCT CTG AAC TAG AGA TTG

ATC AAC GTT ATT GTT TTG GAA TTG AAG GGT TCT GAA ACC
TAG TTG CAA TAA CAA AAC CTT AAC TTC CCA AGA CTT TGG

ACT TTC ATG TGT GAA TAC GCT GAC GAA ACC GCT ACC ATT
TGA AAG TAC ACA CTT ATG CGA CTG CTT TGG CGA TGG TAA

GTT GAA TTT TTG AAC AGA TGG ATT ACC TTC TGT CAA TCT
CAA CTT AAA AAC TTG TCT ACC TAA TGG AAG ACA GTT AGA

ATC ATT TCT ACT TTG ACT TAA TG
TAG TAA AGA TGA AAC TGA ATT ACAGCT.
```

12. A method according to Claim 3 or 5 wherein said substance having human interleukin 2 (IL2) activity is represented by the following amino acid sequence:

```
1                                               10
ALa Pro Thr Ser Ser Ser Thr Lys Lys Thr Gln Leu Gln
                        20
Leu Glu His Leu Leu Leu Asp Leu Gln Met Ile Leu Asn Gly Ile
    30                                          40
Asn Asn Tyr Lys Asn Pro Lys Leu Thr Arg Met Leu Thr Phe Lys
                        50
Phe Tyr Met Pro Lys Lys Ala Thr Glu Leu Lys His Leu Gln Cys
    60                                          70
Leu Glu Glu Glu Leu Lys Pro Leu Glu Glu Val Leu Asn Leu Ala
                        80
Gln Ser Lys Asn Phe His Leu Arg Pro Arg Asp Leu Ile Ser Asn
    90                                          100
Ile Asn Val Ile Val Leu Glu Leu Lys Gly Ser Glu Thr Thr Phe
                        110
Met Cys Glu Tyr Ala Asp Glu Thr Ala Thr Ile Val Glu Phe Leu
    120                                         130
Asn Arg Trp Ile Thr Phe Cys Gln Ser Ile Ile Ser Thr Leu Thr.
```

13.   A method according to Claim 1 wherein said another promoter is a gene controlling a glycolytic pathway enzyme.

14.   A method according to Claim 13 wherein said promoter is a promoter of a gene controlling glyceraldehyde-3-phosphodehydrogenase (GAP-DH), 3-phosphoglycerokinase (PGK) or hexokinase.

15.   A method according to Claim 1 wherein said another promoter is one of a gene controlling an enzyme inductively produced in yeast.

16.   A method according to Claim 15 wherein said promoter is one of a repressible yeast acid phosphatase (PH05) gene.

17.   A method according to Claim 1 wherein said secretor expression vector is one identified as pYIL241.

18.   A method according to Claim 1 wherein said secretor expression vector is one identified as pYαNE412, pYαNE413, pYαNE414 or pYαNE415.

19.   A method according to Claim 1 wherein the host yeast transformed by the secretor expression vector is an α-mating type of yeast.

20.   A method according to Claim 1 wherein said transformant is one identified as JA221/pYIL241.

21.   A method according to Claim 1 wherein said transformant is one identified as JA221/pYαNE412, JA221/pYαNE413, JA221/pYαNE414 or JA221/pYαNE415.

22.   A method according to Claim 1 wherein said carrier gel is κ-carageenan.

23.   A composition containing a protein having human interleukin 2 (IL2) activity which is produced by the method according to Claim 1.

24.   A composition according to Claim 23 wherein said protein is one which is glycosilated.

25.   A composition according to Claim 23 wherein said protein is one capable of being adsorbed on ConA Sepharose and eluted by α-methylmannoside.

FIG. 1

FIG. 2(A)

0171000

2/20

# FIG. 2(B)

# FIG. 3

```
     Met  Tyr  Gly Gly  Phe   leu  Arg  Lys  tyr  Pro  Lys STOP STOP
├─── FI ───┼──────── F2 ────────┼──────── F3 ──────┼──────── F4 ──→
AGCTATGTATGGCGGTTTCCTTCGTAAGTATCCGAAGTAATAG
   TACATACCGCCAAAGGAAGCATTCATAGGCTTCATTATCCTAG
   ←──── F8 ────┼────── F7 ──────┼──── F6 ──────┼──── F5 ──→
```

a - neo endorphin (aNE) gene

FIG. 4

# FIG. 5

# FIG. 6

EcoRI　　　1　　　　　　　　　　　　　DdeI　　10　　　　　　　　　　　　　　　20

Met Ala Pro Thr Ser Ser Ser Thr Lys Lys Thr Gln Leu Gln Leu Glu His Leu Leu Leu Asp Leu Gln

AATTC ATG GCT CCA ACT TCC TCT TCT ACT AAG AAG ACC CAA TTG CAA TTG GAA CAC TTG TTG TTG GAC TTG CAA

G TAC CGA GGT TGA AGG AGA AGA TGA TTC TTC TGG GTT AAC GTT AAC CTT GTG AAC AAC AAC CTG AAC GTT

　　　　　　　　　　　　　　　　　30　　　　　Hinf I　　　HincⅡ　　　　40 HincⅡ

Met Ile Leu Asn Gly Ile Asn Asn Tyr Lys Asn Pro Lys Leu Thr Arg Met Leu Thr Phe Lys Phe Tyr

ATG ATT TTG AAC GGT ATT AAC AAC TAC AAG AAT CCA AAG TTG ACC AGA ATG TTG ACC TTC AAG TTC TAC

TAC TAA AAC TTG CCA TAA TTG TTG ATG TTC TTA GGT TTC AAC TGG TCT TAC AAC TGG AAG TTC AAG ATG

　　　　　　50 HaeⅢ　　　　　　　　　　　　　　　　60

Met Pro Lys Lys Ala Thr Glu Leu Lys His Leu Gln Cys Leu Glu Glu Glu Leu Lys Pro Leu Glu Glu

ATG CCA AAG AAG GCC ACC GAA TTG AAG CAC TTG CAA TGT TTG GAA GAA GAA TTG AAG CCA TTG GAA GAA

TAC GGT TTC TTC CGG TGG CTT AAC TTC GTG AAC GTT ACA AAC CTT CTT CTT AAC TTC GGT AAC CTT CTT

　　　70　　　　　　　　　　DdeI　　　　　　80　　　　　　　　Mbo I　　　　　90

Val Leu Asn Leu Ala Gln Ser Lys Asn Phe His Leu Arg Pro Arg Asp Leu Ile Ser Asn Ile Asn Val

GTC TTG AAC TTG GCT CAA TCT AAG AAC TTC CAC TTG AGA CCA AGA GAC TTG ATC TCT AAC ATC AAC GTT

CAG AAC TTG AAC CGA GTT AGA TTC TTG AAG GTG AAC TCT GGT TCT CTG AAC TAG AGA TTG TAG TTG CAA

　　　　　　　　　　　　　　　　100　　　　　　　　　　　　　　　110

Ile Val Leu Glu Leu Lys Gly Ser Glu Thr Thr Phe Met Cys Glu Tyr Ala Asp Glu Thr Ala Thr Ile

ATT GTT TTG GAA TTG AAG GGT TCT GAA ACC ACT TTC ATG TGT GAA TAC GCT GAC GAA ACC GCT ACC ATT

TAA CAA AAC CTT AAC TTC CCA AGA CTT TGG TGA AAG TAC ACA CTT ATG CGA CTG CTT TGG CGA TGG TAA

　　　　　　　　120　　　　　　　　　　　　　　　130

Val Glu Phe Leu Asn Arg Trp Ile Thr Phe Cys Gln Ser Ile Ile Ser Thr Leu Thr Stop　　SalI

GTT GAA TTT TTG AAC AGA TGG ATT ACC TTC TGT CAA TCT ATC ATT TCT ACT TTG ACT TAA TG

CAA CTT AAA AAC TTG TCT ACC TAA TGG AAG ACA GTT AGA TAG TAA AGA TGA AAC TGA ATT ACAGCT

## FIG. 7

1) AATTCATGGCTC

2) GAAGTTGGAGCCATG

3) CAACTTCCTCTTCTAC

4) CTTCTTAGTAGAAGAG

5) TAAGAAGACCCAAT

6) TTGCAATTGGGT

7) TGCAATTGGAAC

8) AACAAGTGTTCCAA

9) ACTTGTTGTTGGAC

10) ATTTGCAAGTCCAAC

11) TTGCAAATGATTTTG

12) AATACCGTTCAAAATC

13) AACGGTATTAACAACT

14) ATTCTTGTAGTTGTT

15) ACAAGAATCCAAAGTT

16) CTGGTCAACTTTGG

17) GACCAGAATGTTG

18) TTGAAGGTCAACATT

19) ACCTTCAAGTTCTAC

20) TTGGCATGTAGAAC

21) ATGCCAAAGAAGGC

22) TCGGTGGCCTTCT

23) CACCGAATTGAAGC

24) TGCAAGTGCTTCAAT

25) ACTTGCAATGTTTGG

26) TCTTCTTCCAAACAT

27) AAGAAGAATTGAAGC

28) TCCAATGGCTTCAAT

29) CATTGGAAGAAGTCT

30) AAGTTCAAGACTTCT

31) TGAACTTGGCTCAAT

32) TTCTTAGATTGAGCC

33) CTAAGAACTTCCACT

34) GGTCTCAAGTGGAAG

35) TGAGACCAAGAGAC

36) AGATCAAGTCTCTT

37) TTGATCTCTAACAT

38) AACGTTGATGTTAG

39) CAACGTTATTGTTTT

40) CAATTCCAAAACAAT

41) GGAATTGAAGGGTTC

42) GGTTTCAGAACCCTT

43) TGAAACCACTTTCAT

44) TCACACATGAAAGT

45) GTGTGAATACGCTG

46) GTTTCGTCAGCGTAT

47) ACGAAACCGCTACC

48) TCAACAATGGTAGCG

49) ATTGTTGAATTTTTG

50) CCATCTGTTCAAAAAT

51) AACAGATGGATTACCTT

52) ATTGACAGAAGGTAAT

53) CTGTCAATCTATCATT

54) AAAGTAGAAATGATAG

55) TCTACTTTGACTTAATG

56) TCGACATTAAGTC

# FIG. 8

## LIGATION STRATEGY

T4 Ligase

Phosphorylation

Phosphorylation

T4 Ligase

T4 Ligase

T4 Ligase

Synthetic IL-2 Gene

# FIG.9

11/20

# FIG. 10

FIG. 11

0171000

# FIG. 12

FIG. 13

FIG. 14

FIG. 15

FIG. 16

FIG. 17

Crude : Concentrated (×10) : 6ml was applied
$\phi$ 1.5 × 15 (cm)
3.5ml / 10min

IL2
(cpm×10⁻⁴)

(100 time diluted Sample)

$A_{280}$

Fraction No.

FIG. 18

$\phi$ 1.5 × 15 (cm)

DEAE: fractionated  IL-2 preparation

FIG. 19